# EUROPEAN PATENT APPLICATION

(11) **EP 3 928 771 A1**
(43) Date of publication of application: **29.12.2021**
(21) Application number: 21180535.3
(22) Date of filing: 21.06.2021
(51) Int. Cl.: A61K 9/20, A61K 31/423, A61P 25/08

(54) **PHARMACEUTICAL COMPOSITIONS OF 1,2-BENZISOXAZOLE-3-METHANESULFONAMIDE**

(30) Priority: 23.06.2020 EP 20382545
(71) Applicant: Laboratorios Lesvi, S.L., 08970 Sant Joan Despí - Barcelona (ES)
(72) Inventor: ESCACENA CAMPOS, Alejandro, 08970 SANT JOAN DESPÍ (ES); UBEDA PEREZ, Carmen, 08970 SANT JOAN DESPÍ (ES); DIEZ MARTIN, Ignacio, 08970 SANT JOAN DESPÍ (ES)
(74) Representative: ZBM Patents - Zea, Barlocci & Markvardsen

(57) **Abstract**

The present invention relates to immediate release tablets of 1,2-Benzisoxazole-3-methanesulfonamide, also known as zonisamide; process to prepare them and their use as anticonvulsant.

## Description

This application claims the benefit of European Patent Application EP20382545.0 filed on June 23rd, 2020.

### FIELD OF THE INVENTION

The present invention relates to immediate release tablets of 1,2-Benzisoxazole-3-methanesulfonamide, also known as zonisamide.

### BACKGROUND OF THE INVENTION

Zonisamide is a sodium channel blocker, chemically known as 1,2- benzisoxazole-3-methanesulfonamide:

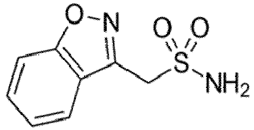

Zonisamide is commercialized as an anticonvulsant for the treatment of epilepsy under the tradename Zonegran^{®}. The Zonegran^{®} zonisamide hard-shelled capsules, currently available in the market from Eisai, are immediate release capsules comprising up to 100 mg of zonisamide.

Zonisamide was first disclosed in US4172896 by Sumitomo Dainippon Pharma. US4172896 also describes pharmaceutical compositions in the form of a tablet comprising 100 mg of zonisamide.

WO2006078948 discloses immediate release capsules comprising about 200 mg of zonisamide.

The recommended dosage regime of zonisamide used as monotherapy corresponds to a usual daily maintenance dose of 300 mg, which can be increased up to 500 mg, being administered once or in two divided doses, when zonisamide is used as adjunctive therapy. However, the higher dose strength of the immediate release Zonegran^{®} zonisamide capsules currently available in the market correspond to 100 mg of zonisamide. To date there is no commercial alternative pharmaceutical formulations comprising zonisamide in dosage amounts as high as the recommended monotherapy dosage of 300 mg per day. It is therefore particularly desirable to provide a stable immediate release tablet of zonisamide capable of having a relatively high dosage amount of active ingredient and being easy and cheap to manufacture.

### SUMMARY

A first aspect of the present invention relates to an immediate release tablet of zonisamide comprising a granulate (i) that contains zonisamide and lactose, an extragranular phase (ii) comprising a diluent other than lactose and optionally a coating (iii). This immediate release tablet of zonisamide is a stable pharmaceutical formulation that can comprise the active substance in a high dosage amount and displays a suitable dissolution profile of said active substance. Additionally, said immediate release tablet of zonisamide shows good cohesiveness and compactability, as well as an appropriate flowability, good distribution and content uniformity.

A second aspect of the present invention is to provide a process for the preparation of the immediate release tablet of zonisamide of the first aspect of the present invention.

A third aspect of the present invention relates to the use of the immediate release tablet of zonisamide of the first aspect of the present invention as an anticonvulsant agent.

### DEFINITIONS

As used herein, the term "immediate release" refers to the rapid release of the majority of the therapeutic compound contained in the tablet, allowing said therapeutic compound to dissolve in the gastrointestinal contents, with no intention of delaying or prolonging the dissolution or absorption of the therapeutic compound. Preferred conditions for immediate release, as states in European Pharmacopoeia (5.17.1, monograph), are the release of at least 80% of the therapeutic compound within a specified time, typically 45 min or less. An equivalent term to immediate release is also the term conventional release.

The term "stable" as used herein, generically refers to the physical and/or chemical stability of the active substance or the composition thereof, during preservation/storage. For instance, compositions which do not show any significant discoloration, which are substantially free of zonisamide decomposition products (the content of zonisamide does not fall below 95% by weight of the initial zonisamide weight content) and whose dissolution rate is preserved during the specified shelf life, particularly during at least 3 months, more particularly during at least 6 months, even more particularly during at least 1 year and most particularly during at least 3 years.

The term "dosage uniformity" as used herein refers to the degree of uniformity in the amount of the active substance among dosage units; defining dosage units as dosage forms containing a single dose or a part of a dose of one active substance in each dosage unit. To ensure the dosage uniformity, each dosage unit should have an active substance content within a narrow range around the label claim. The dosage uniformity of the tablets according to the present invention can be demonstrated by means of performing the method described in European Pharmacopoeia (2.9.40, monograph). As defined therein, the requirements for dosage uniformity are met if the acceptance value (AV) of the dosage units is less than or equal to 15.

The term "dx", as used herein, means that x% of the particles in a composition (based on volume) have a diameter equal to or below a specified d value. Thus, a d90 of 25 µm means that 90% by volume, of the particles, have a diameter equal to or below 25 µm. In addition to using d90 as a measuring reference to determine particle size, d50 can also be used for such purpose. Likewise, a d50 of 10 µm means that 50%, by volume, of the particles, have a diameter equal to or below 10 µm. Also, a d10 of 1 µm means that 10 % of the particle population, by volume, have a diameter of equal to or below 1 µm. It can be determined by laser light diffraction.

The term "granulate" as used herein refers to an active pharmaceutical ingredient and/or excipients present in the tablet forming granules; defining granules as small particles gathered into larger, permanent aggregates in which the original particles can still be identified.

The term "extragranular phase" as used herein refers to the excipients present in the tablet which are out of the granulate.

The term "granulation" as used herein refers to a process to form granules used to improve the handling and manufacturing properties of a formulation, for example by increasing particle size and/or bulk density to improve flow. Various granulation processes are used by those of skill in the art for preparing tablet dosage forms. Examples of such processes include dry granulation and wet granulation. The term "dry granulation" used herein refers to a process to form granules without using any granulation liquid. Dry granulation can be carried out by slugging or by roller compaction processes. Slugging is a process, wherein the material to be granulated is compressed to a large compressed mass, compact, or "slug". Roller compaction is a process wherein the material to be granulated passes between high-pressure rollers that compress it, forming a compact or sheet. The term "wet granulation" used herein refers to a process to form granules by the addition of a granulation liquid (water, organic solvent, or a solution) onto a powder bed which is under the influence of an impeller (in a high-shear granulator), screws (in a twin screw granulator) or air (in a fluidized bed granulator). The agitation resulting in the system along with the wetting of the components within the formulation results in the aggregation of the primary powder particles to produce wet granules, which are subsequently dried.

The term "coating" as used herein refers to adherence, adsorption, loading and/or incorporation, to some extent, preferable uniformly, of at least one solution, dispersion or suspension coating material onto and/or into a substrate. The coating material on the substrate may be of any thickness. Preferably the coating material is a "film coating" that, as used herein, refers to a thin and uniform film applied onto a substrate.

The term "flowability" as used herein refers to the ability of divided solids (for example powders and granules) to flow vertically under defined conditions. The flowability of the immediate release tablets of the present invention has been determined according to the equipment and method described in European Pharmacopeia (2.9.36., monograph).

The term "compactability" as used herein means the ability of the powdered material to be compressed into a tablet of specified strength.

The term "diluent" as used herein refers to an agent able to increase the bulk of the tablet that also may make the tablet easier for the patient and/or a care giver to handle. The diluent may be present in the tablet in the form of a single compound or in the form of a mixture of compounds.

The term "disintegrant" as used herein refers to a facilitator to break up granules or tablets into smaller fragments when getting in contact with liquids to promote rapid drug dissolution. The disintegrant may be present in the tablet in the form of a single compound or in the form of a mixture of compounds.

The term "binder" as used herein refers to an agent able to bind particles which cannot be bound only by a compression force. The binder may be present in the tablet in the form of a single compound or in the form of a mixture of compounds.

The term "wetting agent" (also known as "surfactant") as used herein refers to an agent that increases the spreading and penetrating properties of a liquid by lowering its surface tension, that is the tendency of its molecules to adhere to each other.

The term "glidant" as used herein refers to an agent that has the primary function of enhancing product flow by reducing interparticulate friction. The glidant may be present in the tablet in the form of a single compound or in the form of a mixture of compounds.

The term "lubricant" as used herein refers to an agent able to decrease friction at the interface between a tablet's surface and the die wall during ejection and of reducing wear on punches and dies and of preventing sticking to punch faces. The lubricant may be present in the tablet in the form of a single compound or in the form of a mixture of compounds.

As used herein, the term "% (w/w)" refers to percentage by weight relative to the total weight of the immediate release tablet.

The term "comprising" as used herein encompasses the case of "consisting of".

For the purposes of the invention, the expressions "obtainable", "obtained", "prepared" and similar equivalent expressions are used interchangeably and, in any case, the expression "obtainable" encompasses the expression "obtained".

### DETAILED DESCRIPTION OF THE INVENTION

A first aspect of the present invention relates to an immediate release tablet of zonisamide comprising:
(i) a granulate comprising zonisamide and lactose,
(ii) an extragranular phase comprising at least a diluent other than lactose,
(iii) optionally a coating.

In one embodiment of the first aspect of the present invention, the zonisamide is characterised by a particle size distribution with d10 below 20 µm and/or d50 below 100 µm and/or d90 below 200 µm. Preferably, the d10 is below 10 µm and/or d50 below 50 µm and/or d90 below 100 µm. More preferably, the d10 is below 6 µm and/or d50 below 30 µm and/or d90 below 70 µm. In another embodiment the zonisamide is preferably characterised by a particle size distribution with d10 between 1 µm and 15 µm and/or d50 between 5 µm and 80 µm and/or d90 between 15 µm and 150 µm.

In another embodiment of the first aspect of the present invention, the zonisamide is in an amount from 10% to 80% by weight of the total weight of the tablet, preferably the zonisamide is in an amount from 20% to 60% by weight of the total weight of the tablet, more preferably the zonisamide is in an amount from 35% to 50%.

The immediate release tablet of the first aspect of the present invention allows comprising a wide range of the amount of the active pharmaceutical ingredient, zonisamide. Indeed, the immediate release tablet of the first aspect of the present invention can comprise zonisamide in a dosage amount as high as 300 mg, which allows the patient to take only one single tablet per day instead of the three currently commercially available Zonegran^{®} 100 mg capsules.

In another embodiment of the first aspect of the present invention, the lactose is in any of its various forms, anhydrous, monohydrate, agglomerated forms or atomised forms; preferably the lactose is in its anhydrous or monohydrate forms, more preferably the lactose is as monohydrate lactose. Suitable amount of lactose is from 8% to 40% by weight of the total weight of the tablet, preferably from 10% to 30% by weight of the total weight of the tablet, more preferably from 12% to 20% by weight of the total weight of the tablet.

In a preferred embodiment of the first aspect of the present invention, the lactose is monohydrate lactose and is in an amount from 8% to 40% by weight of the total weight of the tablet, preferably from 10% to 30% by weight of the total weight of the tablet, more preferably from 12% to 20% by weight of the total weight of the tablet. In another preferred embodiment the granulate (i) comprises zonisamide and lactose in a weight ratio from 1:0.1 to 1:1 (w/w), preferably from 1:0.2 to 1:0.8 (w/w), more preferably from 1:0.3 to 1:0.5 (w/w). In a more preferred embodiment, the lactose is monohydrate lactose and is in an amount from 12% to 20% by weight of the total weight of the tablet. In another more preferred embodiment, the lactose is monohydrate lactose and the weight ratio between zonisamide and lactose monohydrate is from 1:0.3 to 1:0.5 (w/w).

The inventors have found that immediate release tablets of zonisamide comprising lactose in the granulate (i) show a flowability that is highly appropriate for pharmaceutical processing.

In another embodiment of the first aspect of the present invention, the granulate (i) further comprises one or more of the following pharmaceutical acceptable excipients: further diluents other than lactose, disintegrants and binders.

Examples of further diluents other than lactose in the granulate (i) include, but are not limited to microcrystalline cellulose, silicified microcrystalline cellulose, dibasic calcium phosphate, maize starch, pregelatinized starch, mannitol, sorbitol, xylitol, glucose, sucrose, maltose, isomaltose and mixtures thereof. The further diluent other than lactose may be preferably selected from microcrystalline cellulose, silicified microcrystalline cellulose, dibasic calcium phosphate and mixtures thereof; more preferably the further diluent other than lactose is microcrystalline cellulose. The amount of further diluent other than lactose may be from 10% to 50% by weight of the total weight of the tablet, preferably from 14% to 40% by weight of the total weight of the tablet, more preferably from 18% to 30% by weight of the total weight of the tablet. In a preferred embodiment the granulate (i) comprises zonisamide and a further diluent other than lactose in a weight ratio from 1:0.1 to 1:1.5 (w/w), preferably from 1:0.3 to 1:1 (w/w), more preferably from 1:0.5 to 1:0.8 (w/w). In a more preferred embodiment, the further diluent other than lactose is microcrystalline cellulose and is in an amount is from 18% to 30% by weight of the total weight of the tablet. In another more preferred embodiment, the further diluent other than lactose is microcrystalline cellulose and the weight ratio between zonisamide and microcrystalline cellulose is from 1:0.5 to 1:0.8 (w/w).

The inventors have surprisingly found that when the granulate (i), comprising zonisamide and lactose, also contains other diluent different from lactose, there is an improvement in the compactability of the granulate (i).

In a preferred embodiment of the first aspect of the present invention the granulate (i) comprises lactose and a further diluent other than lactose in a weight ratio from 1:1 to 1:3 (w/w), preferably from 1:1.3 to 1:2.5 (w/w), more preferably from 1:1.4 to 1:2 (w/w). In another preferred embodiment the granulate (i) comprises zonisamide, lactose and a further diluent other than lactose in a weight ratio from 1:0.1:0.1 to 1:1:1.5 (w/w), preferably from 1:0.2:0.3 to 1:0.8:1 (w/w), more preferably from 1:0.3:0.5 to 1:0.5:0.8 (w/w). It has been found that said ratios provide an appropriate balance between compactability and flowability. In a more preferred embodiment the granulate (i) comprises zonisamide, lactose and microcrystalline cellulose in a weight ratio from 1:0.3:0.5 to 1:0.5:0.8 (w/w).

Examples of disintegrants in the granulate (i) include, but are not limited to sodium croscarmellose, crospovidone, low-substituted hydroxypropyl cellulose, pregelatinized starch, sodium carboxymethyl starch, soy polysaccharides and mixtures thereof. The disintegrant may be preferably selected from sodium croscarmellose, crospovidone, sodium carboxymethyl starch and mixtures thereof; more preferably the disintegrant is sodium croscarmellose. The amount of disintegrant may be from 0.5% to 8% by weight of the total weight of the tablet, preferably from 1% to 5% by weight of the total weight of the tablet, more preferably from 1.5% to 4% by weight of the total weight of the tablet. In a preferred embodiment, the disintegrant is sodium croscarmellose and is in an amount from 1.5% to 4% by weight of the total weight of the tablet.

The use of a disintegrant as one of the pharmaceutical acceptable excipients of the granulate (i) ultimately provides a more rapid release of the zonisamide from the tablet than when disintegrant is not used.

Examples of binders in the granulate (i) include, but are not limited to polyvinylpyrrolidone (povidone), copovidone, pregelatinized starch, methylcellulose, hypromellose, hydroxypropyl cellulose, low-substituted hydroxypropyl cellulose and mixtures thereof. The binder may be preferably selected from povidone, copovidone, pregelatinized starch and mixtures thereof; more preferably the binder is povidone. The amount of binder may be from 0.5% to 15% by weight of the total weight of the tablet, preferably from 1% to 10%, more preferably from 1.5% to 8% by weight of the total weight of the tablet. In a preferred embodiment, the binder is povidone and is in an amount from 1.5% to 8% by weight of the total weight of the tablet.

In another embodiment of the first aspect of the present invention, the granulate (i) is prepared by either dry granulation or wet granulation. In certain embodiments of the present invention, wet granulation is preferred since it is a robust process that provides a stable immediate release tablet of zonisamide showing good cohesiveness and compactability, as well as an appropriate flowability, good distribution and content uniformity. Wet granulation process also aids to improve the dissolution rate of the active substance zonisamide.

Thus, in a preferred embodiment of the first aspect of the present invention, the granulate (i) is obtainable by a wet granulation process. In a more preferred embodiment, the wet granulation process is carried out with a granulate solution comprising water, at least a wetting agent and optionally at least a binder. Examples of wetting agents of the granulation solution include, but are not limited to sodium lauryl sulfate, hypromellose, polyoxyethylene alkyl ethers, polyoxyethylene castor oil derivatives, polyoxyethylene stearates, poloxamer or mixtures thereof; preferably the wetting agent of the granulate solution may be selected from sodium lauryl sulfate, hypromellose, polyoxyethylene stearates or mixtures thereof; more preferably the wetting agent of the granulate solution is sodium lauryl sulfate. The amount of wetting agent of the granulation solution may be from 0.5% to 15% by weight of the total weight of the tablet, preferably from 1% to 10%, more preferably from 2% to 8% by weight of the total weight of the tablet. In a preferred embodiment, the wetting agent is sodium lauryl sulfate and is in an amount from 2% to 8% by weight of the total weight of the tablet. The binder and the amounts of binder may be as defined herein.

In a particular embodiment of the first aspect of the present invention the amount of water of the granulate solution may be below 50% by weight of the total tablet weight, preferably below 40% by weight of the total tablet weight, more preferably below 35% by weight of the total tablet weight. In another particular embodiment, the amount of water of the granulate solution may be between 10% and 45% by weight of the total tablet weight.

In another particular embodiment of the first aspect of the present invention the granulate solution used in the wet granulation process comprise wetting agent and water in a weight ratio from 1:5 to 1:10 (w/w), preferably from 1:6 to 1:9 (w/w), more preferably from 1:7 to 1:8.5 (w/w). It has been found that said ratio provides an appropriate balance between compactability and dissolution profile.

The inventors have also found that when the granulate (i) is prepared by wet granulation using a non-toxic granulation solution comprising water and at least a wetting agent as disclosed above, said granulation solution is easily removed by drying. Moreover, the use of a wetting agent in the aqueous granulation solution allows improving the solubility of the very slightly soluble active substance zonisamide.

The use of a binder as one of the pharmaceutical acceptable excipients of the granulate (i) and/or the granulation solution helps to hold the zonisamide and the excipients together in a cohesive mix, thus creating a more effective and predictable granule formation.

In another embodiment of the first aspect of the present invention, the immediate release tablet of zonisamide comprises an extragranular phase (ii) containing a diluent other than lactose, which may be selected, but not being limited to microcrystalline cellulose, silicified microcrystalline cellulose, maize starch, pregelatinized starch, mannitol, sorbitol, xylitol, glucose, dextrose, sucrose, maltose, isomaltose and mixtures thereof; preferably the diluent other than lactose may be selected from microcrystalline cellulose, silicified microcrystalline cellulose, dibasic calcium phosphate and mixtures thereof; more preferably the diluent other than lactose is microcrystalline cellulose. Additionally, said diluent other than lactose may be in an amount from 2% to 30% by weight of the total weight of the tablet, preferably in an amount from 5% to 20% by weight of the total weight of the tablet, more preferably in an amount from 8% to 15% by weight of the total weight of the tablet. In a preferred embodiment, the diluent other than lactose is microcrystalline cellulose and is in an amount from 8% to 15% by weight of the total weight of the tablet.

The inventors have found that when a diluent different from lactose is used in the extragranular phase (ii), a suitable dissolution profile of the zonisamide, similar to the dissolution profile of zonisamide contained in commercial Zonegran^{®} capsules, is achieved. Thus, the immediate release tablet of zonisamide of the first aspect of the present invention corresponds to a tablet, wherein at least 80% of zonisamide (with respect to the initial zonisamide weight content) is released within 45 min or less. The dissolution profile was determined using a basket apparatus at 100 rpm or a paddle apparatus at 50 rpm, placing the pharmaceutical composition according to the present invention within a vessel containing 900 mL of HCI 0.1N at 37°C.

In another embodiment of the first aspect of the present invention, the extragranular phase (ii) further comprises one or more of the following pharmaceutical acceptable excipients: disintegrants, glidants and lubricants.

Examples of disintegrants in the extragranular phase (ii) include, but are not limited to sodium croscarmellose, crospovidone, low-substituted hydroxypropyl cellulose, pregelatinized starch, sodium carboxymethyl starch, soy polysaccharides and mixtures thereof. The disintegrant may be preferably selected from sodium croscarmellose, crospovidone, sodium carboxymethyl starch and mixtures thereof; more preferably the disintegrant is sodium croscarmellose. The amount of disintegrant may be from 0.5% to 8% by weight of the total weight of the tablet, preferably from 1% to 5% by weight of the total weight of the tablet, more preferably from 1.5% to 4% by weight of the total weight of the tablet. In a preferred embodiment, the disintegrant is sodium croscarmellose and the amount is from 1.5% to 4% by weight of the total weight of the tablet.

In a preferred embodiment a disintegrant is used in the extragranular phase (ii), which improves the disintegrating effect of the disintegrant used in the granulate (i). In a more preferred embodiment the disintegrant used in the extragranular phase (ii) is the same as the disintegrant used in the granulate (i).

Examples of glidants in the extragranular phase (ii) include, but are not limited to anhydrous colloidal silica, talc, calcium phosphate tribasic, magnesium oxide, magnesium silicate, magnesium trisilicate and mixtures thereof; preferably the glidant may be selected from anhydrous colloidal silica, talc, calcium phosphate tribasic and mixtures thereof; more preferably the glidant is anhydrous colloidal silica. The amount of glidant may be from 0.05% to 5% by weight of the total weight of the tablet, preferably from 0.1% to 4% by weight of the total weight of the tablet, more preferably from 0.2% to 2% by weight of the total weight of the tablet. In a preferred embodiment, the glidant is anhydrous colloidal silica and the amount is from 0.2% to 2% by weight of the total weight of the tablet.

The use of a glidant in the extragranular phase (ii) helps to improve the flowability of the powder of the granulate (i).

Examples of lubricants in the extragranular phase (ii) include, but are not limited to magnesium stearate, zinc stearate, calcium stearate, stearic acid, sodium benzoate, glyceryl behenate, hydrogenated castor oil, hydrogenated vegetable oil, sodium stearyl fumarate, talc and mixtures thereof; preferably the lubricant may be selected from magnesium stearate, stearic acid, hydrogenated vegetable oil and mixtures thereof; more preferably the lubricant is magnesium stearate. The amount of lubricant may be from 0.05% to 5% by weight of the total weight of the tablet, preferably from 0.1% to 4%, more preferably from 0.2% to 2% by weight of the total weight of the tablet. In a preferred embodiment, the lubricant is magnesium stearate and the amount is from 0.2% to 2% by weight of the total weight of the tablet.

The use of a lubricant in the extragranular phase (ii) avoids the sticking of the material to the equipment used to make the compression.

In another embodiment of the first aspect of the present invention, the immediate release tablet may comprise a coating (iii), which is preferably a film coating. Said film coating can be prepared in-house or being a commercially available pre-formulated coating material. Said film coating may comprise a film forming agent, which can be any polymer appropriate for film coating. Examples of appropriate polymers/film forming agents include, but are not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose); acrylics such as methacrylate; or methyl methacrylate copolymers; and vinyls such a as polyvinyl alcohol. Preferably, the film forming agent, contained in the coating (iii), is a cellulose derivative or a vinyl.

The coating (iii) of the immediate release tablet further comprises a diluent and a plasticiser. The diluent may be preferably selected from lactose, fructose, polydextrose, sacarose, maltose and mixtures thereof; more preferably the diluent is polydextrose, lactose or mixtures thereof. The plasticiser may be preferably selected from citrate esters (e.g. triethylcitrate, triacetin); low molecular weight polyalkylene oxides (e.g. polyethylene glycols, polypropylene glycols, polyethylene/propylene glycols); glycerol, pentaerythritol, glycerol monoacetate, diacetate or triacetate, propylene glycol, and sodium diethyl sulfosuccinate; more preferably, the plasticiser is polyethylene glycol. The coating (iii) of the immediate release tablet further comprise opacifiers and colorants selected from titanium dioxide, calcium carbonate, iron oxides, aluminium lakes or mixtures thereof.

Said coating (iii) may be used in order to further prevent loss of the active ingredient zonisamide and/or to improve the appearance of the immediate release tablet according to the first aspect of the present invention.

The immediate release tablet of zonisamide according to the first aspect of the present invention is a stable pharmaceutical formulation that can comprise the active substance zonisamide in a dosage amount as high as 300 mg and displays a suitable dissolution profile of said active substance, which is similar to the dissolution profile of zonisamide contained in commercial Zonegran^{®} capsules. Additionally, said immediate release tablet of zonisamide shows good cohesiveness and compactability, as well as an appropriate flowability, good distribution and content uniformity. Thus, the different samples assay carried out on the content is between 95 and 105% of the theoretical content and/or the dosage uniformity is characterized by a good acceptance value equal or lower than 15.

In a particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); and from 8% to 40% (w/w) of lactose, preferably from 10% to 30% (w/w), more preferably from 12% to 20% (w/w),
(ii) an extragranular phase comprising from 2% to 30% (w/w) of a diluent other than lactose, preferably from 5% to 20% (w/w), more preferably from 8% to 15% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); and from 0.5% to 8% (w/w) of a disintegrant, preferably from 1% to 5% (w/w), more preferably from 1.5% to 4% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); and from 0.05% to 5% (w/w) of a lubricant, preferably from 0.1% to 4%, (w/w), more preferably from 0.2% to 2% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); from 0.5% to 8% (w/w) of a disintegrant, preferably from 1% to 5% (w/w), more preferably from 1.5% to 4% (w/w); and from 0.5% to 15% (w/w) of a wetting agent, preferably from 1% to 10% (w/w), more preferably from 2% to 8% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); and from 0.05% to 5% (w/w) of a lubricant, preferably from 0.1% to 4%, (w/w), more preferably from 0.2% to 2% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); and from 0.5% to 8% (w/w) of a disintegrant, preferably from 1% to 5% (w/w), more preferably from 1.5% to 4% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); from 0.05% to 5% (w/w) of a lubricant, preferably from 0.1% to 4%, (w/w), more preferably from 0.2% to 2% (w/w); and from 0.05% to 5% (w/w) of a glidant, preferably from 0.1% to 4%, (w/w), more preferably from 0.2% to 2% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); from 0.5% to 8% (w/w) of a disintegrant, preferably from 1% to 5% (w/w), more preferably from 1.5% to 4% (w/w); and from 0.5% to 15% (w/w) of a wetting agent, preferably from 1% to 10% (w/w), more preferably from 2% to 8% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); and from 0.05% to 5% (w/w) of a lubricant, preferably from 0.1% to 4%, (w/w), more preferably from 0.2% to 2% (w/w); and from 0.05% to 5% (w/w) of a glidant, preferably from 0.1% to 4%, (w/w), more preferably from 0.2% to 2% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); from 0.5% to 8% (w/w) of a disintegrant, preferably from 1% to 5% (w/w), more preferably from 1.5% to 4% (w/w); and from 0.5% to 15% (w/w) of a binder, preferably from 1% to 10% (w/w), more preferably from 1.5% to 8% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); and from 0.05% to 5% (w/w) of a lubricant, preferably from 0.1% to 4%, (w/w), more preferably from 0.2% to 2% (w/w); and from 0.05% to 5% (w/w) of a glidant, preferably from 0.1% to 4%, (w/w), more preferably from 0.2% to 2% (w/w),
(iii) optionally a coating
wherein %(w/w) refers to percentage by weight relative to the total weight of the immediate release tablet.

In another particular embodiment of the first aspect of the present invention, the immediate release tablet of zonisamide according to the first aspect of the present invention comprises:
(i) a granulate comprising from 10% to 80% (w/w) of zonisamide, preferably from 20% to 60% (w/w), more preferably from 35% to 50% (w/w); from 8% to 40% (w/w) of lactose, preferably from 10% to 30% (w/w), more preferably from 12% to 20% (w/w); from 10% to 50% (w/w) of a further diluent other than lactose, preferably from 14% to 40% (w/w), more preferably from 18% to 30% (w/w); from 0.5% to 8% (w/w) of a disintegrant, preferably from 1% to 5% (w/w), more preferably from 1.5% to 4% (w/w); from 0.5% to 15% (w/w) of a wetting agent, preferably from 1% to 10% (w/w), more preferably from 2% to 8% (w/w); and from 0.5% to 15% (w/w) of a binder, preferably from 1% to 10% (w/w), more preferably from 1.5% to 8% (w/w),
(ii) an extragranular phase comprising from 2% to 30% (w/w) of a diluent other than lactose, preferably from 5% to 20% (w/w), more preferably from 8% to 15% (w/w); from 0.5% to 8% (w/w) of a disintegrant, preferably from 1% to 5% (w/w), more preferably from 1.5% to 4% (w/w); and from 0.05% to 5% (w/w) of a lubricant, preferably from 0.1% to 4%, (w/w), more preferably from 0.2% to 2% (w/w); and from 0.05% to 5% (w/w) of a glidant, preferably from 0.1% to 4%, (w/w), more preferably from 0.2% to 2% (w/w),
(iii) optionally a coating
wherein %(w/w) refers to percentage by weight relative to the total weight of the immediate release tablet.

In a more preferred embodiment of the first aspect of the present invention the immediate release tablet of zonisamide comprises:
(i) a granulate comprising zonisamide, lactose and a disintegrant,
(ii) an extragranular phase comprising a diluent other than lactose, a disintegrant and a lubricant,
(iii) optionally a coating.

In a more preferred embodiment of the first aspect of the present invention the immediate release tablet of zonisamide comprises:
(i) a granulate comprising zonisamide, lactose, a further diluent other than lactose and a disintegrant,
(ii) an extragranular phase comprising a diluent other than lactose, a disintegrant and a lubricant,
(iii) optionally a coating.

In a more preferred embodiment of the first aspect of the present invention the immediate release tablet of zonisamide comprises:
(i) a granulate comprising zonisamide, lactose, a further diluent other than lactose, a disintegrant and a wetting agent,
(ii) an extragranular phase comprising a diluent other than lactose, a disintegrant and a lubricant,
(iii) optionally a coating.

In a more preferred embodiment of the first aspect of the present invention the immediate release tablet of zonisamide comprises:
(i) a granulate comprising zonisamide, lactose, a further diluent other than lactose and a disintegrant,
(ii) an extragranular phase comprising a diluent other than lactose, a disintegrant, a lubricant and a glidant,
(iii) optionally a coating.

In a more preferred embodiment of the first aspect of the present invention the immediate release tablet of zonisamide comprises:
(i) a granulate comprising zonisamide, lactose, a further diluent other than lactose, a disintegrant and a binder,
(ii) an extragranular phase comprising a diluent other than lactose, a disintegrant and a lubricant,
(iii) optionally a coating.

In a more preferred embodiment of the first aspect of the present invention the immediate release tablet of zonisamide comprises:
(i) a granulate comprising zonisamide, lactose, a further diluent other than lactose, a disintegrant, a binder and a wetting agent,
(ii) an extragranular phase comprising a diluent other than lactose, a disintegrant and a lubricant,
(iii) optionally a coating.

In a more preferred embodiment of the first aspect of the present invention the immediate release tablet of zonisamide comprises:
(i) a granulate comprising zonisamide, lactose, a diluent other than lactose, a disintegrant and a binder,
(ii) an extragranular phase comprising a diluent other than lactose, a disintegrant, a lubricant and a glidant,
(iii) optionally a coating.

In a more preferred embodiment of the first aspect of the present invention the immediate release tablet of zonisamide comprises:
(i) a granulate comprising zonisamide, lactose, a diluent other than lactose, a disintegrant, a binder and a wetting agent,
(ii) an extragranular phase comprising a diluent other than lactose, disintegrant, a lubricant and a glidant,
(iii) optionally a coating.

In a more preferred embodiment of the first aspect of the present invention the immediate release tablet of zonisamide consists of:
(i) a granulate comprising zonisamide, lactose, a further diluent other than lactose, a disintegrant, a binder and a wetting agent,
(ii) an extragranular phase comprising a diluent other than lactose, a disintegrant, a glidant and a lubricant.

In a more preferred embodiment of the first aspect of the present invention the immediate release tablet of zonisamide comprises:
(i) a granulate comprising zonisamide in an amount from 35% to 50% by weight of the total weight of the tablet and lactose in an amount from 12% to 20% by weight of the total weight of the tablet,
(ii) an extragranular phase comprising a diluent other than lactose in an amount from 8% to 15% by weight of the total weight of the tablet,
(iii) optionally a coating.

In a more preferred embodiment of the first aspect of the present invention the immediate release tablet of zonisamide comprises:
(i) a granulate comprising zonisamide in an amount from 35% to 50% by weight of the total weight of the tablet, lactose in an amount from 12% to 20% by weight of the total weight of the tablet and a disintegrant in an amount from 1.5% to 4% by weight of the total weight of the tablet,
(ii) an extragranular phase comprising a diluent other than lactose in an amount from 8% to 15% by weight of the total weight of the tablet, a disintegrant in an amount from 1.5% to 4% by weight of the total weight of the tablet and a lubricant in an amount from 0.2% to 2% by weight of the total weight of the tablet,
(iii) optionally a coating.

In a more preferred embodiment of the first aspect of the present invention the immediate release tablet of zonisamide comprises:
(i) a granulate comprising zonisamide in an amount from 35% to 50% by weight of the total weight of the tablet, lactose in an amount from 12% to 20% by weight of the total weight of the tablet, a further diluent other than lactose in an amount from 18% to 30% by weight of the total weight of the tablet, and a disintegrant in an amount from 1.5% to 4% by weight of the total weight of the tablet,
(ii) an extragranular phase comprising a diluent other than lactose in an amount from 8% to 15% by weight of the total weight of the tablet, a disintegrant in an amount from 1.5% to 4% by weight of the total weight of the tablet, and a lubricant in an amount from 0.2% to 2% by weight of the total weight of the tablet,
(iii) optionally a coating.

In a more preferred embodiment of the first aspect of the present invention the immediate release tablet of zonisamide comprises:
(i) a granulate comprising zonisamide in an amount from 35% to 50% by weight of the total weight of the tablet, lactose in an amount from 12% to 20% by weight of the total weight of the tablet, a further diluent other than lactose in an amount from 18% to 30% by weight of the total weight of the tablet, and a disintegrant in an amount from 1.5% to 4% by weight of the total weight of the tablet,
(ii) an extragranular phase comprising a diluent other than lactose in an amount from 8% to 15% by weight of the total weight of the tablet, a disintegrant in an amount from 1.5% to 4% by weight of the total weight of the tablet, a lubricant in an amount from 0.2% to 2% by weight of the total weight of the tablet, and a glidant in an amount from 0.2% to 2% by weight of the total weight of the tablet,
(iii) optionally a coating.

In a more preferred embodiment of the first aspect of the present invention the immediate release tablet of zonisamide comprises:
(i) a granulate comprising zonisamide in an amount from 35% to 50% by weight of the total weight of the tablet, lactose in an amount from 12% to 20% by weight of the total weight of the tablet, a further diluent other than lactose in an amount from 18% to 30% by weight of the total weight of the tablet, a disintegrant in an amount from 1.5% to 4% by weight of the total weight of the tablet, and a binder in an amount from 1.5% to 8% by weight of the total weight of the tablet,
(ii) an extragranular phase comprising a diluent other than lactose in an amount from 8% to 15% by weight of the total weight of the tablet, a disintegrant in an amount from 1.5% to 4% by weight of the total weight of the tablet, and a lubricant in an amount from 0.2% to 2% by weight of the total weight of the tablet,
(iii) optionally a coating.

In a more preferred embodiment of the first aspect of the present invention the immediate release tablet of zonisamide comprises:
(i) a granulate comprising zonisamide in an amount from 35% to 50% by weight of the total weight of the tablet, lactose in an amount from 12% to 20% by weight of the total weight of the tablet, a further diluent other than lactose in an amount from 18% to 30% by weight of the total weight of the tablet, a disintegrant in an amount from 1.5% to 4% by weight of the total weight of the tablet, and a binder in an amount from 1.5% to 8% by weight of the total weight of the tablet,
(ii) an extragranular phase comprising a diluent other than lactose in an amount from 8% to 15% by weight of the total weight of the tablet, a disintegrant in an amount from 1.5% to 4% by weight of the total weight of the tablet, a lubricant in an amount from 0.2% to 2% by weight of the total weight of the tablet, and a glidant in an amount from 0.2% to 2% by weight of the total weight of the tablet,
(iii) optionally a coating.

In a more preferred embodiment of the first aspect of the present invention the immediate release tablet of zonisamide consists of:
(i) a granulate comprising zonisamide in an amount from 35% to 50% by weight of the total weight of the tablet, lactose in an amount from 12% to 20% by weight of the total weight of the tablet, a further diluent other than lactose in an amount from 18% to 30% by weight of the total weight of the tablet, a disintegrant in an amount from 1.5% to 4% by weight of the total weight of the tablet, a binder in an amount from 1.5% to 8% by weight of the total weight of the tablet, and a wetting agent in an amount from 2% to 8% by weight of the total weight of the tablet.
(ii) an extragranular phase comprising a diluent other than lactose in an amount from 8% to 15% by weight of the total weight of the tablet, a disintegrant in an amount from 1.5% to 4% by weight of the total weight of the tablet, a lubricant in an amount from 0.2% to 2% by weight of the total weight of the tablet, and a glidant in an amount from 0.2% to 2% by weight of the total weight of the tablet.

A second aspect of the present invention is to provide a process for the preparation of the immediate release tablet of zonisamide of the first aspect by a granulation method, which allows obtaining a tablet that is stable and can comprise the active substance zonisamide in a dosage amount as high as 300 mg and displays a suitable dissolution profile of said active substance, which is similar to the dissolution profile of zonisamide contained in commercial Zonegran^{®} capsules.

Thus, the preparation of the immediate release tablet of zonisamide, described in the first aspect, is carried out by a process that comprises the following steps:
1) providing a granulate (i) which comprises blending zonisamide and lactose,
2) mixing the granulate obtained in step (1) with at least a diluent other than lactose,
3) compressing the blend obtained in step (2) to form a tablet;
4) optionally, coating the tablet obtained in step (3).

All the embodiments described for the first aspect of the present invention also applies to the second aspect of the present invention.

Additionally, in a preferred embodiment of the second aspect of the present invention, the step (1) further comprises adding a granulation solution. The granulation solution is prepared by dissolving at least a wetting agent in water. The wetting agent and the amounts of wetting agent may be as defined herein.

In another preferred embodiment of the second aspect of the present invention, the granulation solution may be prepared by dissolving at least a wetting gent and at least a binder in water. The wetting agent, the binder and the amounts of both, wetting agent and binder, may be as defined herein. When a granulation solution is added in the step (1), the granulate (i) obtained is optionally dried prior to the step (2).

In another embodiment of the second aspect of the present invention, the step (2) further comprises mixing the granulate (i) obtained in step (1) with one or more of the following pharmaceutical acceptable excipients: disintegrant, glidant and lubricant. The disintegrant, the glidant, the lubricant and its amounts may be as disclosed herein.

In a preferred embodiment the disintegrant used in the step (2) is the same as the used in the step (1). In another preferred embodiment the amount of disintegrant used in the step (2) is the same as the used in the step (1). The inventors have found than when a disintegrant is used in both, step (1) and step (2), there is an improvement in the disintegrating effect.

In another embodiment of the second aspect of the present invention the coating carried out in step (4) is preferably film-coating.

A third aspect of the present invention relates to the use of the immediate release tablet of zonisamide of the first aspect of the present invention as an anticonvulsant agent.

The present invention also provides the immediate release tablet of zonisamide of the first aspect of the present invention for use in the treatment of epilepsy.

It also forms part of the invention the use of zonisamide for the manufacture of the immediate release tablet of the first aspect of the present invention for the treatment of epilepsy.

The present invention also provides a method for the treatment of epilepsy comprising administering to a subject in need thereof the immediate release tablet of zonisamide of the first aspect of the present invention.

It will be readily apparent to one skilled in the art that varying substitutions and compositions may be made to the invention disclosed herein without departing from the scope and spirit of the invention. Thus, it should be understood that although the present invention has been specifically disclosed by preferred embodiments and optional features, modifications and variations of the concepts herein disclosed may be resorted to by those skilled in the art, and that such modifications and variations are considered to be falling within the scope of the invention.

The present invention is further illustrated by the following working examples. They should in no case be interpreted as a limitation of the scope of the invention as defined in the claims. Unless indicated otherwise, all indications of percentage are by weight and temperatures are in degrees Celsius.

### EXAMPLES

### Example 1

**Tablet composition**

| **Ingredients** | **mg/Tablet** |
|---|---|
| **Zonisamide¹** | **300.0** |
| Microcrystalline cellulose PH-101 | 154.5 |
| Lactose monohydrate (intragr.) | 106.8 |
| Sodium croscarmellose (intragr.) | 18.0 |
| Povidone | 16.2 |
| Sodium laurylsulfate | 27.3 |
| Microcrystalline cellulose PH-102 (extragr.) | 72.0 |
| Sodium croscarmellose (extragr.) | 18.0 |
| Colloidal anhydrous silica | 3.6 |
| Magnesium stearate | 3.6 |
| Purified water² | 227.1 |
| Total tablet core | 720.0 |

| | |
|---|---|
| ¹Particle size of d₉₀ = 44 µm ²Eliminated during manufacturing | |

### Manufacturing process

1. Zonisamide, microcrystalline cellulose PH-101, lactose monohydrate and sodium croscarmellose (intragranular) are blended during 10 minutes in a high-shear mixer granulator.
2. The granulation solution is prepared, dissolving the povidone and sodium laurylsulfate in purified water.
3. The blend is granulated using the granulation solution.
4. The granulate is dried in a fluid bed dryer.
5. Dried granulate is screened (mesh size 1.2 mm).
6. Cellulose microcrystalline PH-102, sodium croscarmellose (extragranular) and colloidal anhydrous silica are sieved (mesh size 0.5 mm) and blended with the screened granules obtained in step 5 for 10 minutes.
7. Magnesium stearate is sieved (mesh size 0.32 mm) and blended with the blend obtained in step 6 for 5 minutes.
8. The blend obtained in step 7 is compressed to provide a tablet.

### Flowability: Fair

### Dissolution:

**Method: Basket apparatus, 100 rpm**

| *Zonisamide 300 mg tablets* | | | *Zonegran*^{®} *100 mg, 3 capsules* | |
|---|---|---|---|---|
| Time (min) | Mean (%) | | Time (min) | Mean (%) |
| 0 | 0 | | 0 | 0 |
| 5 | 35 | | 5 | 57 |
| 10 | 52 | | 10 | 70 |
| 15 | 58 | | 15 | 74 |
| 20 | 62 | | 20 | 75 |
| 30 | 68 | | 30 | 78 |
| 45 | 73 | | 45 | 80 |

**Method: Paddle apparatus, 50 rpm**

| *Zonisamide 300 mg tablets* | | | *Zonegran*^{®} *100 mg, 3 capsules* | |
|---|---|---|---|---|
| Time (min) | Mean (%) | | Time (min) | Mean (%) |
| 0 | 0 | | 0 | 0 |
| 5 | 30 | | 5 | 46 |
| 10 | 57 | | 10 | 64 |
| 15 | 68 | | 15 | 68 |
| 20 | 74 | | 20 | 71 |
| 30 | 81 | | 30 | 75 |
| 45 | 86 | | 45 | 79 |

### Stability:

The tablets, obtained by means of the manufacturing process described above, were packed in polyvinylchloride-aluminium foil blisters and stored at 25°C ± 2°C temperature and 60 % ± 5% relative humid (RH), or at 40°C ± 2°C temperature and 75 % ± 5% RH for 3 and 6 months, according to the pharmaceutical guideline ICH Q1A (R2) "Stability Testing of New Drug Substances and Products" of February 2003. Results of the stability studies are shown below:

| | Initially | 3 months 25°C/60% HR | 6 months 25°C/60% HR | 3 months 40°C/75% HR | 6 months 40°C/75% HR |
|---|---|---|---|---|---|
| **Assay (%)** | 97.3 | 98.8 | 99.2 | 99.0 | 98.9 |
| **Dissolution (% at 45 min)** | 86 | 83 | 82 | 82 | 85 |

| **Impurities** | | | | | |
|---|---|---|---|---|---|
| Impurity A (%) | <0.1 | <0.1 | <0.1 | <0.1 | <0.1 |
| Total degradation products (%) | <0.1 | <0.1 | <0.1 | <0.1 | <0.1 |

### Example 2

**Tablet composition**

| **Ingredients** | **mg/Tablet** |
|---|---|
| **Zonisamide¹** | **100.0** |
| Microcrystalline cellulose PH-101 | 51.5 |
| Lactose monohydrate (intragr.) | 35.6 |
| Sodium croscarmellose (intragr.) | 6.0 |
| Povidone | 5.4 |
| Sodium laurilsulfate | 9.1 |
| Microcrystalline cellulose PH-102 (extragr.) | 24.0 |
| Sodium croscarmellose (extragr.) | 6.0 |
| Colloidal anhydrous silica | 1.2 |
| Magnesium stearate | 1.2 |
| Purified water² | 68.1 |
| Total tablet core | 240.0 |

| | |
|---|---|
| ¹Particle size of d₉₀ = 44 µm ²Eliminated during manufacturing | |

### Manufacturing process

1. Zonisamide, microcrystalline cellulose PH-101, lactose monohydrate and sodium croscarmellose (intragranular) are blended during 10 minutes in a high-shear mixer granulator.
2. The granulation solution is prepared, dissolving the povidone and sodium laurylsulfate in purified water.
3. The blend is granulated using the granulation solution.
4. The granulate is dried in a fluid bed dryer.
5. Dried granulate is screened (mesh size 1.2 mm).
6. Cellulose microcrystalline PH-102, sodium croscarmellose (extragranular) and colloidal anhydrous silica are sieved (mesh size 0.5 mm) and blended with the screened granules obtained in step 5 for 10 minutes.
7. Magnesium stearate is sieved (mesh size 0.32 mm) and blended with the blend obtained in step 6 for 5 minutes.
8. The blend obtained in step 7 is compressed to provide a tablet.

### Flowability: Good

### Dissolution:

**Method: Basket apparatus, 100 rpm**

| *Zonisamide 100 mg tablets* | |
|---|---|
| Time (min) | Mean (%) |
| 0 | 0 |
| 5 | 60 |
| 10 | 74 |
| 15 | 78 |
| 20 | 80 |
| 30 | 83 |
| 45 | 87 |

| *Zonegran*^{®} *100 mg capsules* | |
|---|---|
| Time (min) | Mean (%) |
| 0 | 0 |
| 5 | 63 |
| 10 | 76 |
| 15 | 78 |
| 20 | 79 |
| 30 | 81 |
| 45 | 83 |

### Example 3

**Tablet composition**

| **Ingredients** | **mg/Tablet** |
|---|---|
| **Zonisamide¹** | **300.0** |
| Microcrystalline cellulose PH-101 | 154.5 |
| Lactose monohydrate (intragr.) | 106.8 |
| Sodium croscarmellose (intragr.) | 18.0 |
| Povidone | 16.2 |
| Sodium laurilsulfate | 27.3 |
| Microcrystalline cellulose PH-102 (extragr.) | 72.0 |
| Sodium croscarmellose (extragr.) | 18.0 |
| Colloidal anhydrous silica | 3.6 |
| Magnesium stearate | 3.6 |
| Purified water² | 215.7 |
| Total tablet core | 720.0 |

| | |
|---|---|
| ¹Particle size of d₉₀ = 44 µm ²Eliminated during manufacturing | |

### Manufacturing process

1. Zonisamide, microcrystalline cellulose PH-101, lactose monohydrate and sodium croscarmellose (intragranular) are blended during 10 minutes in a high-shear mixer granulator.
2. The granulation solution is prepared, dissolving the povidone and sodium laurylsulfate in purified water.
3. The blend is granulated using the granulation solution.
4. The granulate is dried in a fluid bed dryer.
5. Dried granulate is screened (mesh size 1.2 mm).
6. Cellulose microcrystalline PH-102, sodium croscarmellose (extragranular) and colloidal anhydrous silica are sieved (mesh size 0.5 mm) and blended with the screened granules obtained in step 5 for 10 minutes.
7. Magnesium stearate is sieved (mesh size 0.32 mm) and blended with the blend obtained in step 6 for 5 minutes.
8. The blend obtained in step 7 is compressed to provide a tablet.

### Flowability: Good

### Dissolution:

**Method: Paddle apparatus, 50 rpm**

| *Zonisamide 300 mg tablets* | |
|---|---|
| Time | Mean |
| 0 | 0 |
| 5 | 37 |
| 10 | 58 |
| 15 | 67 |
| 20 | 73 |
| 30 | 79 |
| 45 | 83 |

| *Zonegran*^{®} *100 mg, 3 capsules* | |
|---|---|
| Time | Mean |
| 0 | 0 |
| 5 | 49 |
| 10 | 63 |
| 15 | 67 |
| 20 | 69 |
| 30 | 74 |
| 45 | 77 |

### Stability:

The tablets, obtained by means of the manufacturing process described above, were packed in polyvinylchloride-aluminium foil blisters and stored at 25°C ± 2°C temperature and 60 % ± 5% relative humid (RH), or at 40°C ± 2°C temperature and 75 % ± 5% RH for 3 and 6 months, according to the pharmaceutical guideline ICH Q1A (R2) "Stability Testing of New Drug Substances and Products" of February 2003.

**Results of the stability studies are shown below:**

| | Initially | 3 months 25°C/60% HR | 3 months 40°C/75% HR |
|---|---|---|---|
| **Assay (%)** | 99.5 | 99.1 | 99.2 |
| **Dissolution (% at 45 min)** | 83 | 78 | 76 |

| **Impurities** | | | |
|---|---|---|---|
| Impurity A (%) | <0.1 | <0.1 | <0.1 |
| Total degradation products (%) | <0.1 | <0.1 | <0.1 |

## Claims

1. An immediate release tablet of zonisamide comprising:
(i) a granulate comprising zonisamide and lactose,
(ii) an extragranular phase comprising at least a diluent other than lactose,
(iii) optionally a coating.

2. The immediate release tablet of zonisamide according to claim 1, wherein the zonisamide is in an amount from 10% to 80% by weight of the total weight of the tablet, preferably the zonisamide is in an amount from 20% to 60% by weight of the total weight of the tablet, more preferably the zonisamide is in an amount from 35% to 50% by weight of the total weight of the tablet.

3. The immediate release tablet of zonisamide according to any of claims 1 or 2, wherein the lactose is in an amount from 8% to 40% by weight of the total weight of the tablet, preferably the lactose is in an amount from 10% to 30% by weight of the total weight of the tablet, more preferably the lactose is in an amount from 12% to 20% by weight of the total weight of the tablet.

4. The immediate release tablet of zonisamide according to any of claims 1 to 3, wherein the granulate (i) further comprises at least a further diluent other than lactose and/or at least a disintegrant and/or at least a binder and/or at least a wetting agent.

5. The immediate release tablet of zonisamide according to claim 4, wherein the granulate (i) further comprises at least a further diluent other than lactose, which is selected from the group consisting of microcrystalline cellulose, silicified microcrystalline cellulose, dibasic calcium phosphate, maize starch, pregelatinized starch, mannitol, sorbitol, xylitol, glucose, sucrose, maltose, isomaltose and mixtures thereof; preferably microcrystalline cellulose, silicified microcrystalline cellulose, dibasic calcium phosphate and mixtures thereof; more preferably microcrystalline cellulose.

6. The immediate release tablet of zonisamide according to any of claims 4 or 5, wherein the granulate (i) further comprises at least a further diluent other than lactose in an amount from 10% to 50% by weight of the total weight of the tablet, preferably in an amount from 14% to 40% by weight of the total weight of the tablet, more preferably in an amount from 18% to 30% by weight of the total weight of the tablet.

7. The immediate release tablet of zonisamide according to any of claims 4 to 6, wherein the granulate (i) further comprises at least a disintegrant, which is selected from the group consisting of sodium croscarmellose, crospovidone, low-substituted hydroxypropyl cellulose, pregelatinized starch, sodium carboxymethyl starch, soy polysaccharides and mixtures thereof; preferably sodium croscarmellose, crospovidone, sodium carboxymethyl starch and mixtures thereof; more preferably sodium croscarmellose.

8. The immediate release tablet of zonisamide according to any of claims 4 to 7, wherein the granulate (i) further comprises at least a binder, which is selected from the group consisting of polyvinylpyrrolidone (povidone), copovidone, pregelatinized starch, methylcellulose, hypromellose, hydroxypropyl cellulose, low-substituted hydroxypropyl cellulose and mixtures thereof; preferably povidone, copovidone, pregelatinized starch and mixtures thereof; more preferably povidone.

9. The immediate release tablet of zonisamide according to any of claims 4 to 8, wherein the granulate (i) further comprises at least a wetting agent, which is selected from the group consisting of sodium lauryl sulfate, hypromellose, polyoxyethylene alkyl ethers, polyoxyethylene castor oil derivatives, polyoxyethylene stearates, poloxamer or mixtures thereof; preferably sodium lauryl sulfate, hypromellose, polyoxyethylene stearates or mixtures thereof; more preferably sodium lauryl sulfate.

10. The immediate release tablet of zonisamide according to any of claims 1 to 9, wherein the diluent other than lactose of the extragranular phase (ii) is selected from the group consisting of microcrystalline cellulose, silicified microcrystalline cellulose, maize starch, pregelatinized starch, mannitol, sorbitol, xylitol, glucose, dextrose, sucrose, maltose, isomaltose and mixtures thereof; preferably microcrystalline cellulose, silicified microcrystalline cellulose, dibasic calcium phosphate and mixtures thereof; more preferably microcrystalline cellulose.

11. The immediate release tablet of zonisamide according to any of claims 1 to 10, wherein the extragranular phase (ii) further comprises at least a disintegrant and/or at least a glidant and/or at least a lubricant.

12. The immediate release tablet of zonisamide according to the preceding claim 11, wherein the extragranular phase (ii) further comprises at least a disintegrant, which is selected from sodium croscarmellose, crospovidone, low-substituted hydroxypropyl cellulose, pregelatinized starch, sodium carboxymethyl starch, soy polysaccharides and mixtures thereof; preferably sodium croscarmellose, crospovidone, sodium carboxymethyl starch and mixtures thereof; more preferably sodium croscarmellose.

13. The immediate release tablet of zonisamide according to any of claims 1 to 12, wherein the granulate (i) is obtainable by a wet granulation process.

14. A process for the preparation of the immediate release tablet of zonisamide, as defined in claim 1, comprising the steps of:
1) providing a granulate (i) which comprises blending zonisamide and lactose,
2) mixing the granulate (i) obtained in step (1) with at least a diluent other than lactose,
3) compressing the blend obtained in step (2) to form a tablet;
4) optionally, coating the tablet obtained in step (3)

15. The immediate release tablet of zonisamide according to any of claims 1 to 13 for use in the treatment of epilepsy.
